# EUROPÄISCHE PATENTANMELDUNG

(11) **EP 4 691 542 A2**
(43) Veröffentlichungstag der Anmeldung: **11.02.2026**
(21) Anmeldenummer: 25169906.2
(22) Anmeldetag: 14.02.2013
(51) Int. Cl.: A61M 60/416

(54) **INTRAVASALE BLUTPUMPE**

(30) Priorität: 16.02.2012 DE 102012202411
(62) Teilanmeldung aus: 21175160.7
(71) Anmelder: Abiomed Europe GmbH, 52074 Aachen (DE)
(72) Erfinder: SPANIER, Gerd, 52074 Aachen (DE); KIRCHHOFF, Frank, 52074 Aachen (DE); SIESS, Thorsten, 52074 Aachen (DE); MICHELS, Dirk, 52074 Aachen (DE)
(74) Vertreter: Klunker IP Patentanwälte PartG mbB

(57) **Zusammenfassung**

Eine intravasale Blutpumpe mit einem Antriebsteil (11), einem proximal am Antriebsteil befestigten Katheter (14) und einem distal am Antriebsteil befestigten Pumpenteil (12) besitzt einen Elektromotor (21), dessen Motorwelle (25) in dem Antriebsteil (11) mit zwei Radialgleitlagern (27, 31) und einem Axialgleitlager (40) gelagert ist. Im Betrieb wird Spülflüssigkeit durch den Lagerspalt des Axialgleitlagers (40) hindurch und weiter durch das Radialgleitlager (31) am distalen Ende des Antriebsteils (11) gefördert. Die Spülflüssigkeit ist hochviskos, beispielsweise 20%-ige Glykoselösung.

## Beschreibung

Die Erfindung betrifft eine intravasale Blutpumpe zur Unterstützung des Blut - kreislaufs im menschlichen oder gegebenenfalls auch tierischen Körpers. Sie wird perkutan beispielsweise in die Femoralarterie eingeführt und durch das Gefäßsystem des Körpers hindurchgeführt, um beispielsweise die Pumparbeit im Herzen zu unterstützen oder zu ersetzen. Die Erfindung betrifft gleicherma - ßen ein eine solche intravasale Blutpumpe umfassendes System sowie ein Ver - fahren zur Unterstützung des Blutkreislaufs unter Verwendung einer solchen in - travasalen Blutpumpe.

Die Anforderungen an derartige Blutpumpen hinsichtlich Einsatzdauer und ge - ringer Größe nehmen ständig zu. Die kleinsten Pumpen dieser Art haben einen Außendurchmesser von etwa 4 mm. Eine weitere Reduzierung des Außen - durchmessers wird unter anderem durch die in der Pumpe zum Einsatz kom - menden Maschinenelemente begrenzt, die nicht in beliebig geringen Größen zur Verfügung stehen. Darüber hinaus sind die Maschinenelemente enormen Belastungen ausgesetzt, denn aufgrund der geringen Baugröße und der doch beträchtlichen zu fördernden Volumenströme im menschlichen Blutkreislauf ar - beiten diese Pumpen mit hohen Drehzahlen von mehreren 10.000 Umdrehun - gen pro Minute. Waren diese Art von Blutpumpen ursprünglich nur zur kurzzei - tigen Herzunterstützung bestimmt, so werden sie zunehmend auch zur Lang - zeittherapie über mehrere Tage bis zu Wochen eingesetzt.

Aus EP 0 961 621 B1 ist eine intravasale Blutpumpe bekannt, die einen An - triebsteil, einen am proximalen Ende des Antriebsteils angeschlossenen Kathe - ter, durch den hindurch Leitungen zur Stromversorgung des Antriebsteils ver - laufen, und einen am distalen Ende des Antriebsteils befestigten Pumpenteil besitzt. Der Antriebsteil umfasst ein Motorgehäuse mit einem darin angeordne - ten Elektromotor, wobei die Motorwelle des Elektromotors distal aus dem An - triebsteil heraus und in den Pumpenteil hineinragt. Der Pumpenteil seinerseits umfasst ein rohrförmiges Pumpengehäuse, in dem ein Flügelrad dreht, welches auf dem aus dem Motorgehäuse herausragenden Ende der Motorwelle sitzt.

Dabei ist die Motorwelle in dem Motorgehäuse in genau zwei Lagern gelagert, die maximal voneinander entfernt sind, um eine schlagfreie zentrisch genaue Führung des Flügelrads im Pumpengehäuse zu gewährleisten. Während für das Lager am proximalen Ende des Motorgehäuses in der Praxis ein Radialkugella - ger eingesetzt wird, ist das flügelradseitige Lager zudem als Wellendichtung ausgebildet, um zu verhindern, dass Blut in das Motorgehäuse eintritt. In der Praxis wird dem Eintritt von Blut in das Motorgehäuse desweiteren dadurch entgegengewirkt, dass eine Spülflüssigkeit durch das Motorgehäuse und das als Wellendichtung ausgebildete flügelradseitige Lager hindurch geleitet wird. Dies geschieht mit einem Spülflüssigkeitsdruck, der über dem anliegenden Blutdruck liegt.

Die intravasale Blutpumpe fördert das Blut normalerweise von distal nach pro - ximal durch das Pumpengehäuse hindurch und am Motorgehäuse vorbei. Auch eine umgekehrte Förderung ist möglich. In beiden Fällen erzeugt das Flügelrad bei der Blutförderung Axialkräfte, die über die Motorwelle auf die Lagerungen übertragen und von dem Radialkugellager aufgenommen werden.

Ausgehend von diesem Stand der Technik ist es die Aufgabe der vorliegenden Erfindung, Maßnahmen vorzuschlagen, wie die Baugröße derartiger intravasa - ler Blutpumpen weiter reduziert und ihre Lebensdauer erhöht werden kann.

Diese Aufgabe wird durch eine intravasale Blutpumpe mit den Merkmalen des Anspruchs 1 gelöst. In davon abhängigen Ansprüchen sind vorteilhafte Weiter - bildungen und Ausgestaltungen der Erfindung angegeben.

Die erfindungsgemäße Blutpumpe zeichnet sich dadurch aus, dass sie mittels eines Axiallagers axial in dem Motorgehäuse gelagert ist, wobei das Axiallager ein Axialgleitlager oder kombiniertes Radial-Axial-Gleitlager ist. Die axialen Kräfte der Motorwelle brauchen somit nicht mehr von dem am proximalen Ende des Motorgehäuses angeordneten Radialkugellager aufgenommen zu werden. Das Radialkugellager kann daher entsprechend kleiner gebaut werden oder durch ein anderes klein bauendes Radiallager, insbesondere ein Radial - gleitlager, ersetzt werden. Dadurch wiederum ist es möglich, Blutpumpen mit einem weiter reduzierten Außendurchmesser zu entwickeln.

Gleichzeitig verlängert sich durch diese Maßnahme die Lebensdauer der Blut - pumpe, weil das Radiallager aufgrund der verringerten axialen Kräfte, zu deren Aufnahme ein Radiallager ohnehin nicht primär bestimmt ist, entlastet ist, so dass es einem geringeren Verschleiß unterliegt.

Alternativ kann die Erfindung in vorhandene Baugrößen integriert werden, um die Lebensdauer zu erhöhen und den Komplexitätsgrad zu reduzieren.

Die auf die Motorwelle wirkenden Axialkräfte sind der Förderrichtung entgegen - gesetzt. Wenn die Blutpumpe dazu eingerichtet ist, alternativ in proximaler Richtung und in distaler Richtung zu fördern, so wirken auf die Motorwelle in dem einen Fall Axialkräfte in distaler Richtung und in dem anderen Fall in proxi - maler Richtung. Bei einer solchen Blutpumpe sind dementsprechend zwei Axi - algleitlager oder Axial-Radial-Gleitlager in dem Motorgehäuse zur axialen Lage - rung der Motorwelle vorzusehen. Das Axialgleitlager kann in einfacher Weise durch eine auf der Motorwelle angeordnete Scheibe gebildet werden, die sich gegen eine umlaufende Schulter des Motorgehäuses abstützt. Im Falle eines Axial-Radial-Gleitlagers besitzt die Scheibe eine konvexe oder konkave, insbe - sondere sphärische, Lagerfläche. Nachfolgend wird der Begriff "Axialgleitlager" synonym für beide Varianten, Axialgleitlager und Radial-Axial-Gleitlager, ver - wendet.

Das Motorgehäuse selbst ist mit einer geeigneten Flüssigkeit gefüllt, die einen Schmierfilm im Lagerspalt des Axialgleitlagers bildet. Alternativ kann durch eine Spülflüssigkeitszuleitung zugeführte Spülflüssigkeit, die das am distalen Ende des Motorgehäuses liegende Radiallager durchströmt, auch den Lager - spalt des Axialgleitlagers durchströmen und auf diese Weise dazu genutzt wer - den, den Schmierfilm im Lagerspalt zu bilden. Um in diesem Fall sicherzustel - len, dass die Spülflüssigkeit das distale Radiallager mit einem Druck erreicht, der über dem anliegenden Blutdruck liegt, kann in zumindest einer der den La - gerspalt des Axialgleitlagers bildenden Oberflächen ein Kanal vorgesehen wer - den, der den Lagerspalt von radial außen nach radial innen durchsetzt, so dass die Spülflüssigkeit durch diesen Kanal zum distalen Radiallager fließen kann. Dieser Kanal muss nicht notwendigerweise in einer Lagerspaltoberfläche lie - gen, sondern kann auch als separater Kanal oder als Bohrung realisiert sein. Den Kanal in einer der Lagerspaltoberflächen vorzusehen hat aber den Vorteil, dass sich der Schmierfilm im Lagerspalt weniger erwärmt, weil ständig ein Teil des Schmierfilms durch nachströmende Spülflüssigkeit ersetzt wird. Vorzugs - weise befindet sich der Kanal in der stationären Lagerspaltoberfläche, um die radiale Förderleistung zu minimieren.

Vorzugsweise ist das Axialgleitlager als hydrodynamisches Gleitlager ausgebil - det. Im Gegensatz zu einem einfachen Gleitlager wird in einem hydrodynami - schen Gleitlager durch Pumpwirkung der beiden relativ zueinander bewegten Oberflächen ein Druck im Schmierfilm aufgebaut. Dazu kann der Lagerspalt ge - mäß einer bevorzugten Variante in Umfangsrichtung des Axialgleitlagers be - reichsweise als konvergierender Spalt ausgebildet sein. Dabei ist vorzugsweise die bewegte Oberfläche eben, das heißt die gegenüberliegende stationäre Oberfläche des Lagerspalts weist Rampen auf, die in Drehrichtung der beweg - ten Oberfläche zur bewegten Oberfläche hin konvergieren. So wird im Lager - spalt ein Keil gebildet, in den die Schmierflüssigkeit hinein transportiert wird, wodurch ein Druck aufgebaut wird, aufgrund dessen sich die bewegte Oberflä - che von der statischen Oberfläche entfernt. In diesem Zustand herrscht Glei - treibung auf einem Flüssigkeitsfilm, die quasi verschleißfrei ist. Da die Blut - pumpe im Normalfall kontinuierlich mit hoher Drehzahl fördert, ist die Blutpum - pe besonders verschleißarm und dementsprechend für Langzeitanwendungen geeignet.

In der einfachsten Ausführungsform kann die bewegte Scheibe als Taumel - scheibe ausgeführt sein und schlicht durch die Schrägstellung den konvergen - ten Spalt bzw. Keil bilden.

Anstelle eines Lagerspalts mit konvergierenden Oberflächenzonen kann eine der den Lagerspalt bildenden Oberflächen eine oder mehrere spiralförmig an - geordnete Rillen aufweisen. In diesem Fall wird die Schmierflüssigkeit durch die Relativbewegung der beiden Oberflächen entlang der Rillen zur Lagermitte ge - pumpt und baut dort einen Druck auf, der wiederum dazu führt, dass sich die beiden Oberflächen voneinander entfernen. Bei der Spiralrillenlagervariante ist es bevorzugt, die spiralförmig angeordneten Rillen in der bewegten Oberfläche vorzusehen, weil so die Schmierflüssigkeitsförderung in die Rillen hinein effek - tiver ist.

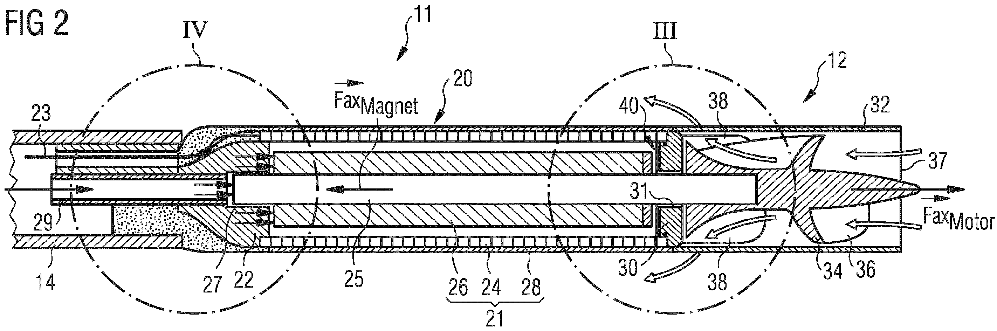

Sollte bauartbedingt der Axialschub (F _{ax Rotor}) der Pumpvorrichtung größer sein als die Tragfähigkeit des Axialgleitlagers, so kann der Axialschub der Pumpvor - richtung durch eine geeignete axiale Anordnung des rotierenden Magneten im Motor teilweise kompensiert werden. Erfindungsgemäß verhält sich der rotie - rende Magnet wie ein Tauchmagnet, der sich magnetisch in der Mitte des stati - schen Motorteils aufhalten will. Wird dieser nunmehr aus dieser Ruhelage her - ausgezogen, so entsteht eine Magnetkraft in umgekehrter Richtung (F _{ax Magnet}). Diese Kraft kann in Richtung des Axialschubs (F _{ax Rotor}) zur weiteren axialen Sta - bilisierung oder in entgegengesetzter Richtung eingesetzt werden und das axiale Gleitlager entlasten. Weiterhin kann durch Variation des Drucks der Spülflüssigkeit die resultierende Kraft auf das Axiallager eingestellt werden (vgl. Fig. 2).

Weiter bevorzugt ist es, wenn die den Lagerspalt des Axialgleitlagers bilden - den Oberflächen aus Keramik bestehen, vorzugsweise Zirkoniumoxid. Aus Ke - ramik lassen sich hochfeste, verschleißarme Oberflächen herstellen. Insbeson - dere kann in einfacher Weise das gesamte distale Ende des Motorgehäuses, einschließlich der Oberfläche für das Axialgleitlager, aus einem einstückigen Keramikteil hergestellt werden, so dass die Gesamtherstellungskosten der Blut - pumpe gering sind.

Die Anschlussdrähte des in dem Motorgehäuse aufgenommenen Elektromotors werden normalerweise um das proximal gelegene Radiallager außen herumge - führt und mit den im Katheter verlaufenden Stromversorgungsleitungen elek - trisch leitend verbunden, insbesondere verlötet. Gemäß einer bevorzugten Ausgestaltung der Erfindung werden die Anschlussdrähte des Elektromotors nun durch den Außenring des Radiallagers hindurch oder vorteilhafterweise in einer oder mehreren radial außen liegenden Nuten des Außenrings geführt. Dadurch wird Bauraum in radialer Richtung gespart, was wiederum positiv ist für die Entwicklung von Blutpumpen mit kleinem Außendurchmesser. So kann beispielsweise Bauraum für eine Druckmessung und deren Durchführung am proximalen Motorende erreicht werden.

Die Anschlussdrähte können vorteilhaft proximal von dem proximal gelegenen Radiallager auf einer Oberfläche des Motorgehäuses mit den Stromversor - gungsleitungen verlötet werden. Dies ist vorteilhaft, weil das direkte elektri - sche Verbinden der Anschlussdrähte des Elektromotors mit den Stromversor - gungsleitungen aufgrund der geringen Dicke der Anschlussdrähte und der ver - gleichsweise großen Dicke der Stromversorgungsleitungen Schwierigkeiten be - reiten kann. Wenn die betreffende Oberfläche des Elektromotors aus einem Kunststoff oder aus Keramik besteht, wie nachfolgend noch erläutert wird, so kann der Lötstellenbereich vor dem Verlöten leitfähig beschichtet werden, bei - spielsweise mit Kupfer, und das Verlöten der Anschlussdrähte und der Versor - gungsleitungen erfolgt jeweils separat in diesem Bereich.

Vorzugsweise wird der betreffende Teil des Motorgehäuses anschließend in Kunststoff eingebettet, wobei die Lötstellen und vorzugsweise auch die Motor - wicklungen mit eingebettet werden, so dass die Lötstellen einerseits elektrisch isoliert und andererseits mechanisch geschützt sind.

Gemäß einer bevorzugten Weiterbildung der Erfindung werden auch die Radi - allager für die Motorwelle am proximalen Ende und am distalen Ende des Mo - torgehäuses jeweils als Gleitlager ausgebildet. Da diese Radiallagerungen im wesentlichen nur dazu dienen, die Welle zentrisch genau zu führen und dementsprechend nur geringe radiale Kräfte aufzunehmen sind, können sie als einfache Gleitlager ausgeführt werden. Ein Radialgleitlager beansprucht deut - lich weniger Bauraum in radialer Richtung als ein Wälzkörperlager mit seinen Innen- und Außenringen. Dies wirkt sich wieder positiv auf die Möglichkeiten aus, Blutpumpen mit kleinem Außendurchmesser herzustellen.

Insbesondere ist es bevorzugt, das am proximalen Ende des Motorgehäuses liegende Radialgleitlager aus Keramik herzustellen, wobei das Keramiklager di - rekt an der Umfangsoberfläche der Motorwelle anliegt. In entsprechender Wei - se kann auch das am distalen Ende des Motorgehäuses liegende Radiallager ausgebildet sein. Die der Keramikoberfläche gegenüberliegende Oberfläche der Motorwelle, welche zusammen mit der Keramikoberfläche den Lagerspalt des Radialgleitlagers bildet, ist vorzugsweise mit einer amorphen Kohlenstoff - beschichtung (DLC = Diamant-Like-Carbon bzw. Diamant-Like-Coating) be - schichtet. DLC-Schichten sind besonders verschleißfest und reibungsarm. Sie sind nur wenige Mikrometer dick und können beispielsweise im chemischen Dampfabscheidungsverfahren (CVD) oder physikalischen Dampfabscheidungs - verfahren (PVD) erzeugt werden. Alternativ kann die Welle aus einer bruchsi - cheren Keramik gefertigt werden.

Im Betrieb wird die Blutpumpe an eine Spülflüssigkeitsquelle angeschlossen und Flüssigkeit durch die Spülflüssigkeitsleitung hindurch ins Motorgehäuse ge - leitet. Die Spülflüssigkeit fließt dann durch das Axialgleitlager und weiter durch das distale Radiallager hindurch. Im Axialgleitlager bildet es den Schmierfilm im Lagerspalt. Der Druck, mit dem die Spülflüssigkeit durch das Motorgehäuse strömt, wirkt sich jedoch negativ auf die Weite des Lagerspalts aus. Denn je hö - her der Spülflüssigkeitsdruck ist, desto geringer wird die Lagerspaltweite und desto dünner ist der Schmierfilm zwischen den Gleitflächen. Je dünner der Schmierfilm ist, desto größer ist wiederum der zur Überwindung der Reibkräfte notwendige Motorstrom zum Antreiben des Elektromotors. Dies ist ungünstig für die Steuerung der Blutpumpe, weil man normalerweise anhand von hinter - legten Kennlinien allein auf Basis des Motorstroms und der Drehzahl (beides bekannte Größen) das aktuelle Fördervolumen ermittelt. Wenn sich der Spül - flüssigkeitsdruck zusätzlich auf den Motorstrom auswirkt, so wäre eine weitere Einflussgröße zu berücksichtigen. In Anbetracht der Tatsache, dass derselbe Blutpumpentyp für unterschiedlichste Anwendungen mit unterschiedlichen Spülflüssigkeitsdrücken zwischen 300 und 1400 mmHg betrieben werden kann, ist es wichtig, eine Abhängigkeit des Motorstroms vom Spülflüssigkeitsdruck zu vermeiden.

Dies kann tatsächlich erreicht werden, wenn als Spülflüssigkeit eine Flüssigkeit mit einer Viskosität gewählt wird, die deutlich höher ist als die Viskosität von Wasser (η = 0,75 mPa • s bei 37°C). Denn mit einer hochviskosen Spülflüssig - keit wird der Flüssigkeitsfilm selbst bei hohen Drücken aufrecht erhalten und die Reibung des Axialgleitlagers ist dementsprechend unabhängig vom Spül - flüssigkeitsdruck. Es hat sich herausgestellt, dass man mit einer Spülflüssig - keit, deren Viskosität bei 37°C ca. 1,2 mPa • s beträgt oder noch darüber liegt, das Axialgleitlager als einfaches Gleitlager ausbilden kann und nicht als hydro - dynamisches Gleitlager auszubilden braucht. Gute Ergebnisse wurden bei - spielsweise mit einer ≥ 20%-igen Glykoselösung zwischen Keramikoberflächen aus Zirkoniumoxid erzielt.

Nachfolgend wird die Erfindung beispielhaft anhand der begleitenden Zeich - nungen erläutert. Darin zeigen:
- Figur 1: eine schematische Darstellung der Einführung einer Blutpumpe vor den linken Ventrikel, mit Positionierung ihrer Ansaugkanüle im linken Ventrikel,
- Figur 2: einen schematischen Längsschnitt eines Ausführungsbeispiels der Blutpumpe,
- Figur 3: eine vergrößerte Darstellung der Einzelheit III aus Figur 2,
- Figur 4: eine Variante zur Einzelheit III aus Figur 3,
- Figur 5: eine vergrößerte Darstellung der Einzelheit IV aus Figur 2,
- Figuren 6A und 6B: eine Axialgleitlageroberfläche in Draufsicht und als Abwicklung ge - mäß einem ersten Ausführungsbeispiel,
- Figur 7: eine Axialgleitlageroberfläche im Querschnitt gemäß einem zweiten Ausführungsbeispiel, und
- Figur 8: eine Axialgleitlageroberfläche in Draufsicht gemäß einem dritten Ausführungsbeispiel.

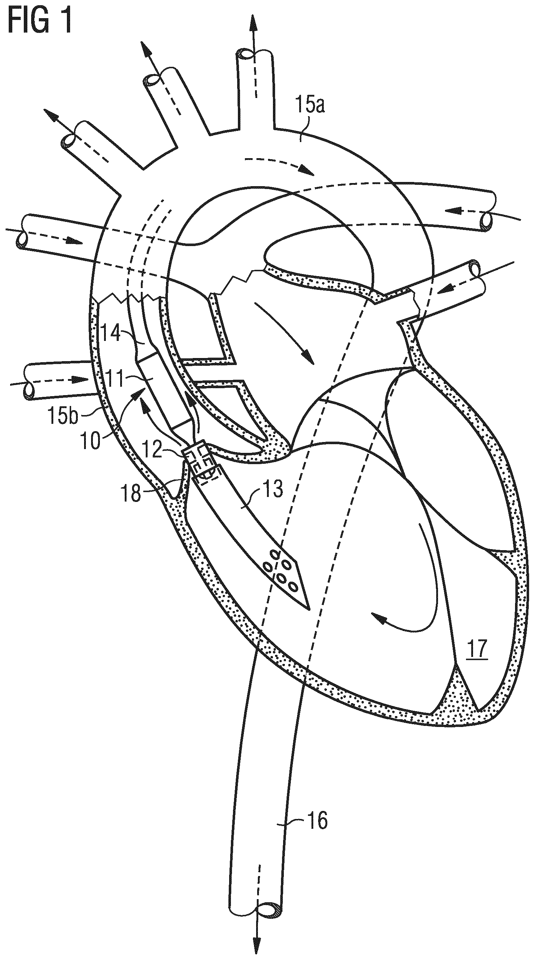

In Figur 1 ist die Verwendung einer Blutpumpe 10 zur Unterstützung des linken Ventrikels dargestellt. Die Blutpumpe weist einen Motorteil 11 und einen Pum - penteil 12 auf, die koaxial hintereinander angeordnet sind und eine stabförmi - ge Bauform ergeben. Der Pumpenteil ist durch einen flexiblen Saugschlauch 13 verlängert, der an seinem Ende und/oder in seiner Seitenwand Öffnungen für den Blutzutritt zur Pumpe aufweist. Das vom Saugschlauch 13 abgewandte Ende der Blutpumpe 10 ist mit einem Katheter 14 verbunden, der durch den Aortenbogen 15a und die Aorta 16 eingeführt wurde. Die Blutpumpe 10 wird so platziert, dass sie hauptsächlich in der aufsteigenden Aorta 15b liegt, der Pum - penteil 12 mit dem Saugschlauch 13 aber im wesentlichen im linken Ventrikel 17 liegt. Die Aortenklappe 18 legt sich im Schließzustand an die Außenseite des Pumpengehäuses oder des Saugschlauchs 13 an. Die Blutpumpe 10 mit dem vorgelagerten Saugschlauch 13 wird durch Vorschieben des Katheters 14 gegebenenfalls unter Verwendung eines Führungsdrahts in die dargestellte Po - sition vorgeschoben. Der Saugschlauch 13 passiert dabei die Aortenklappe 18 retrograd, so dass durch den Saugschlauch 13 Blut angesaugt und in die Aorta 16 gepumpt wird. Insoweit entspricht die Blutpumpe der aus der EP 0 961 621 B1 bekannten Blutpumpe.

Der Einsatz der Blutpumpe ist nicht auf die in Figur 1 dargestellte Anwendung beschränkt, bei der es sich lediglich um ein typisches Anwendungsbeispiel handelt. So kann die Pumpe auch durch andere periphere Gefäße, wie die Arte - ria Subclavia, eingeführt oder auch im rechen Herzen platziert werden.

Figur 2 zeigt ein bevorzugtes Ausführungsbeispiel der Blutpumpe mit dem Mo - torteil 11 und dem damit fest verbundenen Pumpenteil 12. Der Motorteil 11 weist ein langgestrecktes Gehäuse 20 auf, in welchem der Elektromotor 21 un - tergebracht ist. Der Stator 24 des Elektromotors 21 weist in üblicher Weise zahlreiche umfangsmäßig verteilt angeordnete Wicklungen sowie einen ma - gnetischen Rückschluss 28 in Längsrichtung auf. Er ist mit dem Motorgehäuse fest verbunden. Der Stator 24 umgibt den mit der Motorwelle 25 verbundenen Rotor 26, der aus in Wirkrichtung magnetisierten Permanentmagneten besteht. Die Motorwelle 25 erstreckt sich über die gesamte Länge des Motorgehäuses 20 und ragt distal aus diesem heraus. Dort trägt sie ein Flügelrad 34 mit davon abstehenden Flügeln 36 oder Pumpenschaufeln, die in einem rohrförmigen Pumpengehäuse 32 drehen, welches seinerseits fest mit dem Motorgehäuse 20 verbunden ist.

An das proximale Ende des Motorgehäuses 20 schließt sich der flexible Kathe - ter 14 abdichtend an. Durch den Katheter 14 verlaufen elektrische Kabel 23 zur Stromversorgung und Steuerung des Elektromotors 21. Desweiteren verläuft durch den Katheter 14 eine Spülflüssigkeitsleitung 29, welche die proximale Stirnwand 22 des Motorgehäuses 20 durchsetzt. Spülflüssigkeit wird durch die Spülflüssigkeitsleitung 29 ins Innere des Motorgehäuses 20 gespeist und tritt durch die Stirnseite 30 am distalen Ende des Motorgehäuses aus. Der Spül - druck ist so gewählt, dass er über dem anliegenden Blutdruck liegt, um auf diese Weise zu verhindern, dass Blut ins Motorgehäuse eindringt, und liegt je nach Anwendungsfall zwischen 300 und 1400 mmHg.

Bei einer Rotation des Flügelrades 34 wird Blut durch die stirnseitige Ansaug - Öffnung 37 des Pumpengehäuses 32 angesaugt und in axialer Richtung im Pumpengehäuse 32 nach hinten befördert. Durch Austrittsöffnungen 38 im Pumpengehäuse 32 strömt das Blut aus dem Pumpenteil 12 aus und weiter entlang dem Motorgehäuse 20. Hierdurch wird der Abtransport der im Antrieb erzeugten Wärme sichergestellt. Es ist auch möglich, den Pumpenteil mit um - gekehrter Förderrichtung zu betreiben, wobei Blut an dem Motorgehäuse 20 entlang angesaugt wird und aus der Öffnung 37 austritt.

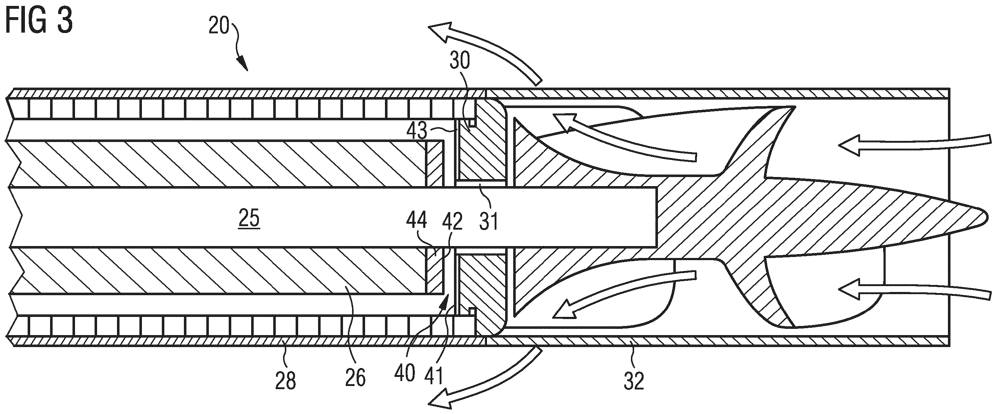

Die Motorwelle 25 ist einerseits am proximalen Ende des Motorgehäuses und andererseits am distalen Ende des Motorgehäuses in Radiallagern 27 und 31 gelagert. Die Radiallager sind in diesem Ausführungsbeispiel jeweils als einfa - che Gleitlager ausgeführt. Darüber hinaus ist die Motorwelle 25 in dem Motor - gehäuse 20 auch axial gelagert. Das Axiallager 40 ist ebenfalls als Gleitlager ausgeführt. Das Axialgleitlager 40 wird nachfolgend in Bezug auf Figur 3 ge - nauer erläutert. Es dient dazu, axiale Kräfte der Motorwelle 25, die in distaler Richtung wirken, wenn das Flügelrad 34 von distal nach proximal fördert, auf - zunehmen. Sollte die Blutpumpe dazu eingesetzt werden, Blut auch oder nur in umgekehrter Richtung zu fördern, so ist ein entsprechendes Axialgleitlager 40 (auch/oder) in entsprechender Weise am proximalen Ende des Motorgehäuses 20 vorzusehen.

Die Blutpumpe gemäß Fig. 2 kann alternativ ohne Spülflüssigkeit für den kurz - zeitigen Einsatz von wenigen Stunden verwendet werden. Für diesen Fall wer - den die Gleitlager einmalig geschmiert, und das distale Gleitlager 31 ist zudem mit einer Radiallippendichtung versehen, um den Eintritt von Blut zu verhin - dern. Auf eine Spülflüssigkeitsleitung kann dann vorteilhaft insgesamt verzich - tet werden.

Figur 3 zeigt den Ausschnitt III aus Figur 2 in größerem Detail. Zu sehen sind insbesondere das Radialgleitlager 31 und das Axialgleitlager 40. Der Lager - spalt des Radialgleitlagers 31 wird gebildet einerseits durch die Umfangsober - fläche der Motorwelle 25, die DLC-beschichtet ist, und andererseits durch die Oberfläche der Durchgangsbohrung in der distalen Stirnwand 30 des Motorge - häuses 20, welche als Keramikteil, zum Beispiel aus Zirkoniumoxid, hergestellt ist.

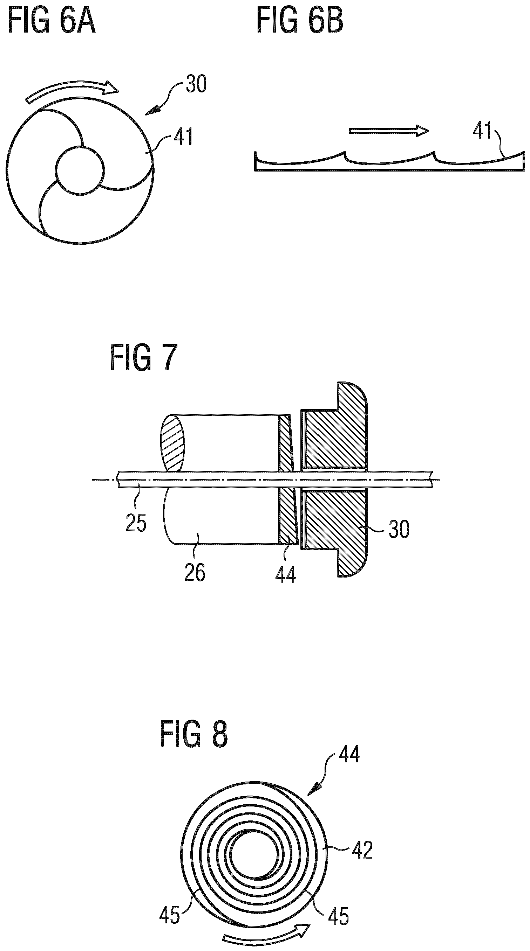

Der Lagerspalt des Axialgleitlagers 40 wird einerseits durch die axial innen lie - gende Oberfläche 41 der Stirnwand 30 und eine ihr gegenüberliegende Ober - fläche 42 gebildet. Diese gegenüberliegende Oberfläche 42 ist Bestandteil ei - ner Keramikscheibe 44, die auf der Motorwelle 25 distal vom Rotor 26 sitzt und sich mit dem Rotor 26 dreht. Ein Kanal 43 in der Lagerspaltoberfläche 41 der Stirnwand 30 stellt sicher, dass Spülflüssigkeit zwischen den Lagerspaltoberflä - chen 41 und 42 des Axialgleitlagers 40 hindurch zum Radialgleitlager 31 flie - ßen und distal aus dem Motorgehäuse 20 austreten kann. Die Spülflüssigkeit wird mit einer Viskosität von mindestens 1,2 mPa • s bei 37°C gewählt. Als geeignet hat sich beispielsweise 20%-Glykoselösung erwiesen. Das in Figur 3 dar - gestellte Axialgleitlager 40 ist ein normales Gleitlager. Hydrostatische Gleitla - gervarianten werden nachfolgend in Bezug auf die Figuren 6A/B, 7 und 8 be - schrieben. Der Axialspalt des Axialgleitlagers 40 ist anders als dargestellt mit wenigen µm sehr klein.

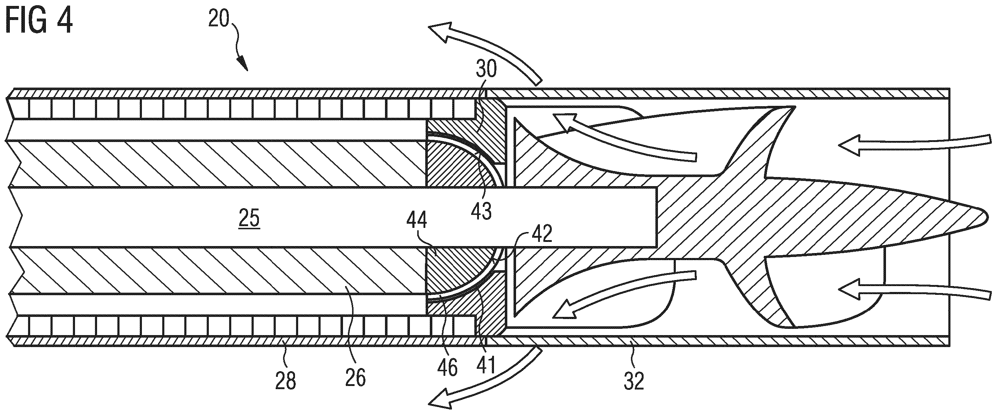

Anstelle des Axialgleitlagers 40 und Radialgleitlagers 31 kann auch ein kombi - niertes Radial-Axial-Gleitlager 46 mit einer konkaven Lagerschale, in der eine konvexe Lagerfläche läuft, realisiert werden. Eine solche Variante ist in Figur 4 anhand eines sphärischen Gleitlagers 46 dargestellt. Die Lagerspaltoberfläche 41 ist sphärisch konkav gestaltet, und die gegenüberliegende Lagerspaltober - fläche 42 ist entsprechend sphärisch konvex ausgebildet. Der Kanal 43 liegt wieder in der ortsfesten Lagerspaltoberfläche 41 der Stirnwand 30. Alternativ kann die feststehende Lagerspaltoberfläche 41 der Stirnwand 30 konvex und die gegenüberliegende Lagerspaltoberfläche 42 konkav ausgebildet werden.

Die Oberflächen 42, 43 können anstatt sphärisch auch konisch sein. Vorzugsweise ist ein entsprechendes Radial-Axial-Gleitlager an beiden Seiten des Mo - torgehäuses 20 vorgesehen, um bei axialem Wandern der Welle 25 keinen ra - dialen Versatz zuzulassen. Der Vorteil eines kombinierten Axial-Radial-Gleitla - gers liegt in der höheren Belastbarkeit. Nachteilig ist hingegen der größere Reibdurchmesser.

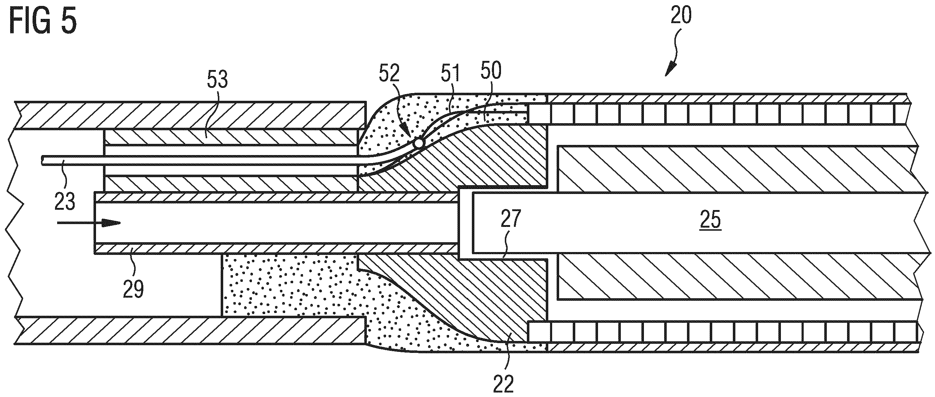

Figur 5 zeigt das Radiallager 27 am distalen Ende des Motorgehäuses 20. Auch hier ist die Motorwelle 25 mit einer DLC-Beschichtung versehen und läuft in ei - ner Lagerbuchse, die einen integralen Bestandteil der wiederum aus Keramik gefertigten proximalen Stirnwand 22 des Motorgehäuses 20 bildet. Insofern entspricht das Radialgleitlager 27 dem Radialgleitlager 31.

Über den Umfang der Stirnwand 22 verteilt sind drei axial verlaufende Nuten 50 im Abstand von 120° vorgesehen, von denen in Figur 2 lediglich eine zu se - hen ist. Durch diese Nuten 50 hindurch führen dünne Anschlussdrähte 51 zu den Wicklungen des Stators 24. Die Anschlussdrähte 51 werden auf der proxi - malen Seite der Stirnwand 20 festgelötet, wobei die Lötstelle 52 zuvor mit ei - ner lokalen Kupferbeschichtung leitfähig gemacht wird. An derselben Lötstelle 52 wird außerdem das Ende der Stromversorgungsleitung 23 verlötet. Die An - bindung der Drähte der Statorwicklungen mit den Stromversorgungsleitungen kann mit allen herkömmlichen Fügeverfahren (Löten, Schweißen, Klemmen, La - serschweißen, Spaltschweißen, Kontaktkleben, etc.) erfolgen. Anschließend wird die Stirnwand 22 einschließlich der Anschlussdrähte 51 und der Lötstellen 52 mit einem Kunststoffmaterial ummantelt, wobei gleichzeitig auch die Motor - wicklungen des Stators mit ummantelt werden. Dies kann beispielhaft in einem Vakuumverguß erfolgen.

Die vorbeschriebene Blutpumpe verzichtet auf Radialkugellager zur Lagerung der Motorwelle 25, welche schwer zu montieren sind und eine Mindestgröße von 3 mm besitzen. Dadurch ist es möglich, Pumpen mit noch kleineren Außen - durchmessern von beispielsweise lediglich 3 mm herzustellen. Außerdem ist die Lebensdauer dieser Blutpumpe gegenüber solchen mit Radialkugellagern aufgrund geringeren Verschleißes deutlich erhöht. Laufzeiten > 30 Tage kön - nen so verschleißarm realisiert werden. Letzteres ist äußerst bedeutsam, da die Lagerung und der schlagfreie Lauf des Flügelrades entscheidend sind für eine geringe Blutschädigung.

Figur 6A zeigt in Aufsicht die Oberfläche 41 der distalen Stirnwand 30 des Mo - torgehäuses 20 gemäß einem alternativen Ausführungsbeispiel. Figur 6B zeigt eine Abwicklung der Oberfläche 41 aus Figur 6A. Die Oberfläche 41 selbst ist stationär. Die durch den Pfeil angedeutete Richtung zeigt an, in welcher Rich - tung sich die gegenüberliegende Oberfläche 42 des Gleitlagers 40 bewegt. Dies entspricht dann auch der Richtung, in der sich der Schmierfilm innerhalb des Lagerspalts relativ zur stationären Oberfläche 41 bewegt. Dementspre - chend besitzt die Oberfläche 41 hintereinander angeordnete Rampen, die zu - sammen mit der gegenüberliegenden bewegten Oberfläche 42, welche eben ist, konvergierende Spalte bilden. Dadurch baut sich im Schmierfilm ein hydro - dynamischer Druck auf, der dafür sorgt, dass die den Lagerspalt bildenden Oberflächen auf Abstand bleiben.

Eine Ausbildung der rotierenden Oberfläche mit den rampenartigen Strukturen gemäß den Figuren 6A und 6B ist zwar für die Leistungsfähigkeit des axialen Gleitlagers von Vorteil, führt aber zu einer erhöhten radialen Förderwirkung im Lagerspalt, die der Förderrichtung der Spülflüssigkeit entgegensteht. In Figur 7 ist die einfachste Form einer rampenartigen Realisierung des konvergenten Spalts in Form einer Taumelscheibe dargestellt. Hierbei wird einfach die Schei - be 44 schrägt eingebaut oder minimal schräg geschliffen. Die Schrägstellung beträgt typischerweise 1 bis 5 µm.

Figur 8 zeigt eine andere Variante für eine hydrodynamisch wirkende Oberflä - che des Axialgleitlagers 40. Es handelt sich um ein sogenanntes Spiralrillenla - ger, welches bevorzugt an der sich bewegenden Oberfläche des Lagerspalts ausgebildet wird, also dementsprechend an der Oberfläche 42 der Keramik - scheibe 44. In diesem Falle sind in der Oberfläche 42 mehrere Rillen 45 spiral - förmig angeordnet. Die Rillen 45 sind in Figur 8 lediglich schematisch angedeu - tet. Wenn die Keramikscheibe 44 in der durch den Pfeil in Figur 8 angedeuteten Richtung dreht, so wird der Schmierfilm entlang der Rillen 45 nach radial innen gefördert und baut dort einen Druck auf, der wiederum dafür sorgt, dass die den Lagerspalt bildenden Oberflächen zueinander auf Abstand gehalten wer - den.

## Patentansprüche

1. Intravasale Blutpumpe (10), umfassend
- einen Antriebsteil (11), der ein Motorgehäuse (20) mit einem proximalen Ende und einem distalen Ende und einen in dem Motorgehäuse angeordneten Elektromotor (21) aufweist, wobei der Elektromotor eine Motorwelle (25) be - sitzt, die mit einem Ende aus dem distalen Ende des Motorgehäuses (20) her - ausragt und in dem Motorgehäuse sowohl am proximalen Ende als auch am distalen Ende des Motorgehäuses radial gelagert (27, 31) ist,
- einen Katheter (14), der mit dem proximalen Ende des Motorgehäuses (20) verbunden ist und entlang dessen Leitungen (23) zur Stromversorgung des Elektromotors verlaufen, und
- einen Pumpenteil (12) mit einem rohrförmigen Pumpengehäuse (32), das am distalen Ende des Motorgehäuses (20) befestigt ist, und einem Flügelrad (34), das auf dem aus dem distalen Ende des Motorgehäuses herausragenden Ende der Motorwelle (25) sitzt und in dem Pumpengehäuse (32) drehbar ist, **dadurch gekennzeichnet, dass** die Motorwelle (25) mittels mindestens einem Axialgleitlager (40) oder Radial-Axial-Gleitlager (46) axial in dem Motorgehäu - se (20) gelagert ist.

2. Intravasale Blutpumpe nach Anspruch 1, wobei das Axialgleitlager (40) oder Radial-Axial-Gleitlager (46) eine auf der Motorwelle (25) angeordnete Scheibe (44) umfasst, die sich gegen eine umlaufende Schulter des Motorge - häuses (20) abstützt.

3. Intravasale Blutpumpe nach einem der Ansprüche 1 oder 2, umfassend eine Spülflüssigkeitsleitung (29), die so angeordnet ist, dass durch die Spülflüs - sigkeitsleitung zugeführte Flüssigkeit den Lagerspalt des Axialgleitlagers (40) und das am distalen Ende des Motorgehäuses liegende Radiallager (31) bzw. den Lagerspalt des Radial-Axial-Gleitlagers (46) durchströmt.

4. Intravasale Blutpumpe nach einem der Ansprüche 1 bis 3, wobei zumindest eine der den Lagerspalt des Axialgleitlagers (40) oder Radial-Axial-Gleitlagers (46) bildenden Oberflächen (41, 42) einen Kanal (43) aufweist, der den Lager - spalt von radial außen nach radial innen durchsetzt.

5. Intravasale Blutpumpe nach einem der Ansprüche 1 bis 4, wobei der Lager - spalt des Axialgleitlagers (40) oder Radial-Axial-Gleitlagers (46) in Umfangs - richtung bereichsweise als konvergierender Spalt ausgebildet ist.

6. Intravasale Blutpumpe nach Anspruch 5, wobei eine bewegte Oberfläche (42) der den Lagerspalt des Axialgleitlagers (40) oder Radial-Axial-Gleitlagers (46) bildenden Oberflächen (42, 41) eben ist.

7. Intravasale Blutpumpe nach einem der Ansprüche 1 bis 4, wobei eine der den Lagerspalt des Axialgleitlagers (40) oder Radial-Axial-Gleitlagers (46) bil - denden Oberflächen (41, 42) eine oder mehrere spiralförmig angeordnete Ril - len (45) aufweist.

8. Intravasale Blutpumpe nach Anspruch 7, wobei eine bewegte Oberfläche (42) der den Lagerspalt des Axialgleitlagers (40) oder Radial-Axial-Gleitlagers (46) bildenden Oberflächen (42, 41) die spiralförmig angeordnete(n) Rille(n) aufweist.

9. Intravasale Blutpumpe nach einem der Ansprüche 1 bis 8, wobei die den Lagerspalt des Axialgleitlagers (40) oder Radial-Axial-Gleitlagers (46) bilden - den Oberflächen (41, 42) aus Keramik bestehen.

10. Intravasale Blutpumpe nach einem der Ansprüche 1 bis 9, wobei das am proximalen Ende des Motorgehäuses (20) gelegene Radiallager (27) einen Au - ßenring aufweist und Anschlussdrähte (51) des Elektromotors (21) durch den Außenring hindurch oder in einer radial außen liegenden Nut (50) des Außen - rings verlaufen.

11. Intravasale Blutpumpe nach Anspruch 10, wobei die den Lagerspalt des Axialgleitlagers (40) oder Radial-Axial-Gleitlagers (46) bildenden Oberflächen (41, 42) aus Keramik bestehen.

12. Intravasale Blutpumpe nach Anspruch 11, wobei das Motorgehäuse zumin - dest teilweise ein gegossenes Kunststoffgehäuse ist, und wobei die Verlötun - gen (52) mit umgossen ist.

13. Intravasale Blutpumpe nach einem der Ansprüche 1 bis 9, wobei zumindest eines der radialen Lagerungen (27, 31) der Motorwelle (25) am proximalen Ende und am distalen Ende des Motorgehäuses (20) als Radialgleitlager ausge - bildet sind.

14. Intravasale Blutpumpe nach Anspruch 13, wobei bei dem zumindest einen Radialgleitlager eine der den Lagerspalt bildenden Oberflächen eine Keramiko - berfläche und die entsprechend andere der den Lagerspalt bildenden Oberflä - chen eine amorphe Kohlenstoffbeschichtung (DLC) ist.

15. System, umfassend die intravasale Blutpumpe nach Anspruch 3 und eine Spülflüssigkeitsquelle zur Versorgung der Spülflüssigkeitsleitung mit einer Flüs - sigkeit, deren Viskosität bei 37°C ≥ 1,2 mPa • s liegt.

16. Verfahren zur Unterstützung eines Blutkreislaufs unter Verwendung der in - travasalen Blutpumpe nach Anspruch 3, wobei der Spülflüssigkeitsleitung Flüs - sigkeit mit einer Viskosität zugeführt wird, die bei 37°C ≥ 1,2 mPa • s liegt.

17. Verfahren nach Anspruch 16, wobei die Spülflüssigkeit eine ≥ 20%-ige Glukoselösung ist.
